# EUROPEAN PATENT SPECIFICATION

(11) **EP 1 450 112 B1**
(45) Date of publication and mention of the grant of the patent: **28.09.2011**
(21) Application number: 02772985.4
(22) Date of filing: 09.10.2002
(51) Int. Cl.: F24F 1/00, F24F 3/147, F24F 6/00, F24F 6/10, B01D 53/26, A61L 9/00, A61L 9/16

(54) **A HUMIDIFYING UNIT OF AN AIR CONDITIONER, AND AN AIR CONDITIONER COMPRISING THE HUMIDIFYING UNIT**
BEFEUCHTUNGSEINHEIT EINER KLIMAANLAGE UND KLIMAANLAGE ENTHALTEND DIE BEFEUCHTUNGSEINHEIT
UNITE D'HUMIDIFICATION DE CONDITIONNEUR D'AIR ET CONDITIONNEUR D'AIR CONTENANT L'UNITE D'HUMIDIFICATION

(30) Priority: 29.11.2001 JP 2001364229
(43) Date of publication of application: 25.08.2004
(73) Proprietor: DAIKIN INDUSTRIES, LTD., Osaka-shi, Osaka 530-8323 (JP)
(72) Inventor: TOKUI, Takashi c/o DAIKIN INDUSTRIES, LTD.,, Kusatsu-shi, Shiga 525-0044 (JP); KUDOU, Hidekazu c/o DAIKIN INDUSTRIES, LTD.,, Kusatsu-shi, Shiga 525-0044 (JP); YOSHINAGA, Kouzou c/o DAIKIN INDUSTRIES, LTD.,, Kusatsu-shi, Shiga 525-0044 (JP)
(74) Representative: HOFFMANN EITLE
(86) International application number: PCT/JP2002/010503
(87) International publication number: WO 2003/048648

(56) References cited:
- EP-A- 0 513 384
- EP-A- 0 810 023
- FR-A- 2 170 993
- JP-A- 9 170 783
- JP-A- 9 170 783
- JP-A- 2000 291 978
- JP-A- 2001 065 922
- JP-A- 2001 263 731
- US-A- 5 733 451

## Description

### TECHNICAL FIELD OF THE INVENTION

The present invention relates to a humidifying unit of an air conditioner.

### RELATED ART

Air conditioners have recently appeared on the market provided with a humidifying unit that takes in moisture from the outdoor air and transfers moisture to an indoor space.

This humidifying unit has a structure like, for example, the one disclosed in Japanese Patent Application Kokai No. 2001-41511. Therein, a humidifying rotor made of, for example, zeolite, is used to humidify air delivered to an indoor space. Specifically, the moisture in the air is adsorbed on the humidifying rotor, and that moisture is then desorbed from the humidifying rotor using a heater. By adding that desorbed moisture to the air delivered to the indoor space, the air delivered to the indoor space is thereby humidified.

Thus, because humidified air can be supplied to the indoor space by an air conditioner provided with a humidifying unit, the indoor relative humidity can be maintained at a comfortable level even, for example, in the winter when the air is dry. In addition, because the abovementioned humidifying unit is provided with a function that supplies air taken in from an outdoor space to the indoor space, it is also possible to perform ventilation by using the function of supplying outdoor air to the indoor space.

The humidifying unit takes in air from the outdoor space, humidifies that air (or does not humidify that air), and then supplies the air to the indoor space. The humidifying unit is also provided with a ventilation function, as described above. Because the outdoor air is generally cleaner than the indoor air, it can be said that the humidifying unit provides greater value than an air conditioner because it delivers outdoor air to the indoor space.

However, if the cleanliness level of the outdoor air is low, or if the outdoor air includes a large amount of automobile exhaust gas because, for example, an arterial road or a high-speed highway is nearby, or if the outdoor air smells from a gutter in which wastewater is flowing, then there is a risk that supplying the outdoor air to the indoor space will unfortunately pollute the indoor air. Accordingly, in such a case, it becomes problematic to run the abovementioned humidifying unit.

Conversely, a simple countermeasure would be to provide the humidifying unit with a filter that adsorbs malodorous or deleterious components. Although it is thereby conceivable to purify the air delivered to the indoor space, this approach unfortunately increases the size of the humidifying unit, as well as creates the trouble of, for example, replacing the filter.

JP 2000-291978 A discloses an adsorbing rotor that supports a deodorizing catalyst that is activated at all times. This document fails to disclose a catalyst being provided as a coating only on a portion of a humidity regulating element facing a beater, with the effectiveness of the catalyst being increased by heating.

An air conditioner is also disclosed in JP 09-170 783 A.

### SUMMARY OF THE INVENTION

According to the present invention, there is provided a humidifying unit of an air conditioner for adding humidified air to the regulated air supplied to an indoor space, comprising; a humidity regulating element monolithically comprising: a moisture adsorbing and desorbing material for adsorbing and desorbing moisture from and to the air, and a catalyst for decreasing polluting components in the air; a heater that is arranged to heat said humidity regulating element; and a fan that is arranged to pass air through said humidity regulating element, wherein said catalyst is provided as a coating only on a portion of said humidity regulating element facing said beater, the effectiveness of said catalyst being increased by heating.

By heating the humidity regulating element with a heater, moisture is desorbed from the humidity regulating element, and that moisture can be added to the air passing through the humidity regulating element by a fan.

Moreover, although the humidity regulating element, which includes a catalyst, has a function that decreases the amount of the polluting component in the air, that function can be produced effectively if the humidity regulating element is heated to a high temperature by a heater.

There are catalysts wherein the value that indicates the effectiveness in decreasing the polluting components (e.g., the rate of decomposition of the polluting components) increases at high temperatures. When such a catalyst is included in the humidity regulating element, it is preferable to heat the catalyst.

In light of the above, the humidifying unit of the present claim is constituted so that the catalyst in the humidity regulating element is coated on a portion near the heater that heats the humidity regulating element (heater side portion). Thereby, the effectiveness of decreasing the polluting components by the catalyst can be increased.

In preferred embodiments, the humidifying unit of the air conditioner is a unit that takes in air from the outdoor space, and supplies that air to the indoor space. In addition, the air conditioner can perform a humidifying operation, wherein humidified air from the humidifying unit is supplied to the indoor space, and an air supplying operation, wherein non-humidified air from the humidifying unit is supplied to the indoor space. Further, the heater executes a first heating control mode for desorbing moisture from the humidity regulating element when in the humidifying operation, and executes a second heating control mode for promoting the function of the catalyst of the humidity regulating element when in the air supplying operation.

Here, it is possible to perform the humidifying operation and the air supplying operation in the air conditioner using the function of the humidifying unit, i.e., to draw in air from the outdoor space and supply that air to the indoor space. It is assumed that the air supplying operation is performed if ventilation of the indoor space is needed but humidification is not needed.

Thus, the heater is controlled so that the humidity regulating function of the humidity regulating element is sufficiently produced when in the humidifying operation (first heating control mode); and the heater is controlled so that the function of the humidity regulating element in decreasing the polluting components is sufficiently produced when in the air supplying operation (second heating control mode). In this manner, the control of the heater is divided into the mode during the humidifying operation and the mode during the air supplying operation, and therefore the effectiveness of the humidity regulating element in the air conditioner can be appropriately produced. Furthermore, it is evident that the polluting components can be decreased during the humidifying operation by the first heating control mode.

In further preferred embodiments, the humidifying unit of the air conditioner is provided in an air conditioner comprising an indoor unit and an outdoor unit. Further, the humidity regulating element is provided in the outdoor unit.

In this case, the humidity regulating element, which includes a photocatalyst, is provided in the outdoor unit, and not the indoor unit. Accordingly, it becomes easier for the prescribed light, included in, e.g., outdoor sunlight, to impinge upon the photocatalyst in the humidity regulating element.

At least a portion of the catalyst may be a photocatalyst. A representative photocatalyst is titanium oxide. Further, there is virtually no decline in the functioning of the titanium oxide as a catalyst, and its effectiveness is maintained semipermanently.

The humidity regulating element to be used is an element monolithically comprising: a moisture adsorbing and desorbing material for adsorbing and desorbing moisture from and to the air; and a catalyst for decreasing polluting components in the air.

The single element includes a moisture adsorbing and desorbing material that can regulate the humidity of the air by adsorbing and desorbing moisture, and a catalyst for decreasing the polluting components in the air. Thus, by using a humidity regulating element that monolithically comprises a moisture adsorbing and desorbing material and a catalyst, the provision of, for example, a separate filter is no longer needed, and the amount of polluting components in the air can be decreased by passing the air through the humidity regulating element, while controlling an increase in the size of the apparatus, such as a dehumidifier, a humidifier, a dehumidifier/humidifier, or an air conditioner.

Furthermore, it is conceivable to use zeolite, silica gel, or alumina as the moisture adsorbing and desorbing material. It is conceivable to use a variety of catalysts, such as a noble metal catalyst, an oxide catalyst, or a photocatalyst, and these may be used individually or in combination. In addition, in a conventional element that uses an adsorbing material like zeolite, a technique is used that impregnates, for example, a ceramic with zeolite. However, it is also acceptable to employ a technique that impregnates a base metal with the catalyst, and it is also acceptable to use a technique that coats the catalyst on at least one portion of the surface of a base metal.

In addition, because the polluting components are not just simply adsorbed, but rather are decreased by the catalyst, there is nearly no deterioration of the catalyst itself nor any unfortunate reduction in effectiveness.

It is contemplated that the catalyst may be a deodorizing catalyst that decomposes an odor component in the air into an odorless component.

In this case, because the catalyst in the humidity regulating element decomposes the odor component in the air into an odorless component, most of the odor component is eliminated from the air that passes through this element. Accordingly, even if using an apparatus that draws in indoor air, once again regulates the humidity of that air, and then supplies that air to the indoor space; or even if using an apparatus that takes in outdoor air, and supplies that air to the indoor space; then the odor component is decreased by passing the air through the element of the present claim, thereby no longer causing discomfort to the user of the apparatus.

Herein, odor components include, for example, methyl sulfide, ammonia, trimethylamine, and acetaldehyde (included in tobacco smoke).

It is conceived for the catalyst to change a deleterious component in the air into an innocuous component.

Here, because the catalyst in the humidity regulating element changes deleterious components in the air into innocuous components, most of the deleterious components are eliminated from the air that passes through this element. Accordingly, even if using an apparatus that takes in the indoor air, once again regulates the humidity of that air, and then supplies that air to the indoor space; and even if using an apparatus that takes in outdoor air and supplies that air to the indoor space; then the deleterious components are decreased by passing the air through the element of the present claim, and thereby the harm to the user of the apparatus from the deleterious component is decreased.

Herein, deleterious components include, for example, dioxin, formaldehyde, as well as NOx, SOx, CO, and HC, which are included in the exhaust gas of automobiles and the like.

It is contemplated that, in embodiments of the invention, the moisture adsorbing and desorbing material is present approximately uniformly over the entirety of the humidity regulating element, and the catalyst is present in a portion of the humidity regulating element.

In this case, it is possible to ensure that the amount of moisture that can be adsorbed and desorbed with respect to the size of the element is large because the moisture adsorbing and desorbing material is present in an approximately uniform manner over the entirety of the element. In addition, it is possible to produce ample effectiveness by concentrating the distribution of the catalyst in an efficient region. For example, if the catalyst is located in a region in which applicable conditions enable the catalyst to function efficiently (a portion of the humidity regulating element), then the polluting component can be sufficiently decreased even if the catalyst is not present over the entirety of the element.

An advantage achievable with embodiments of the present invention is to provide a humidifying unit of an air conditioner that increases the effectiveness of that humidity regulating element, including providing a humidity regulating element that can decrease the amount of pollutants in the air while controlling an increase in the size of the air conditioner.

### BRIEF EXPLANATION OF DRAWINGS

To enable a better understanding of the present invention, and to show how the same may be carried into effect, reference will now be made, by way of example only, to the accompanying drawings, in which:-
FIG. 1 is a perspective view that illustrates the external configuration of the air conditioner according to one embodiment of the present invention.
FIG. 2 is schematic diagram of the refrigerant circuit.
FIG. 3 is an exploded perspective view of the outdoor unit;
FIG. 4(a) is a side-view schematic diagram of the humidifying unit;
FIG. 4(b) is a side-view schematic diagram of the humidifying unit according to another embodiment; and
FIG. 5 is a side-view schematic diagram of the humidifying unit according to another embodiment.

### DETAILED DESCRIPTION

### OVERVIEW CONSTITUTION OF THE AIR CONDITIONER

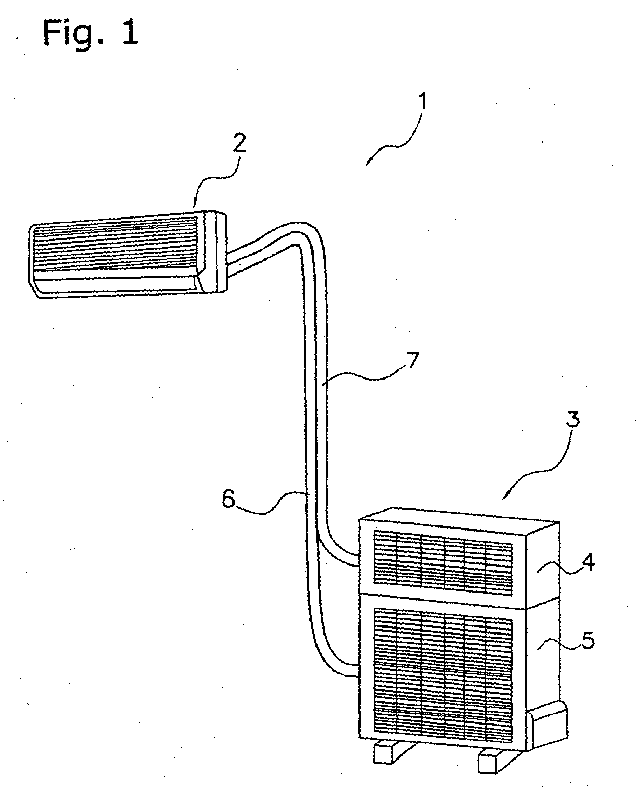

FIG. 1 shows an external view of the air conditioner that uses a humidifying rotor (humidity regulating element) according to one embodiment of the present invention.

An air conditioner 1 comprises an indoor unit 2, which is affixed to, e.g., an indoor wall, and an outdoor unit 3, which is installed in the outdoor space. The outdoor unit 3 comprises an outdoor air conditioning unit 5 that houses, e.g., an outdoor heat exchanger and an outdoor fan; and a humidifying unit 4 that transports humidified air to the indoor unit 2. An indoor heat exchanger is housed in the indoor unit 2, and an outdoor heat exchanger is housed in the outdoor unit 3. Further, the heat exchangers, and a refrigerant passageway 6 that connects these heat exchangers, constitute the refrigerant circuit. In addition, an inlet passageway 7 is provided between the humidifying unit 4 and the indoor unit 2 in order to supply air from the humidifying unit 4 to the indoor unit 2 side.

### CONSTITUTION OF THE REFRIGERANT CIRCUIT

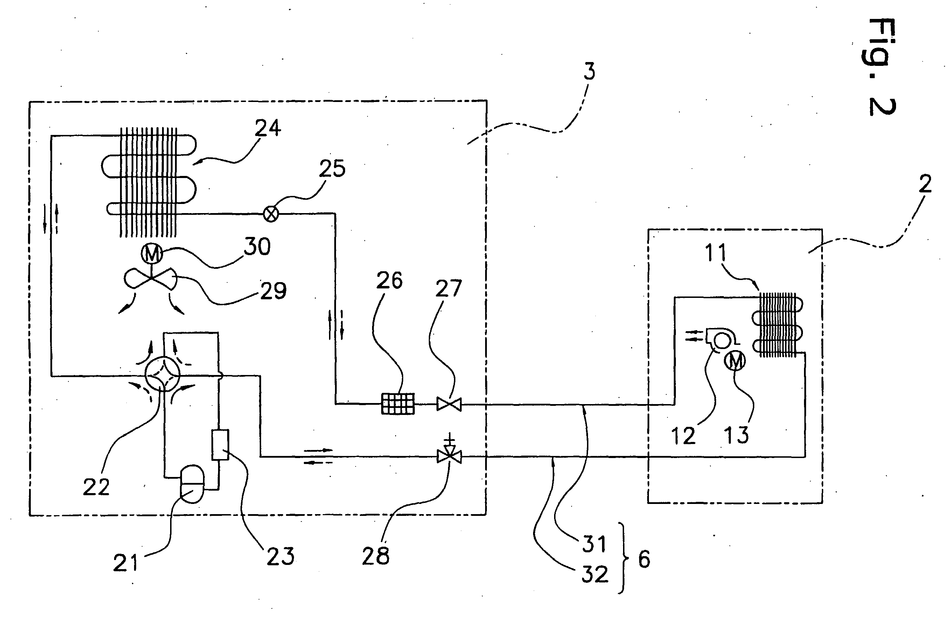

FIG. 2 is a schematic diagram of the refrigerant circuit used by the air conditioner 1.

An indoor heat exchanger 11 is provided in the indoor unit 2. This indoor heat exchanger 11 comprises a heat transfer tube that is folded multiple times at both ends in the longitudinal direction, and a plurality of fans that are inserted in the heat conducting tube, and heat is exchanged between the indoor heat exchanger 11 and the air that contacts the indoor heat exchanger 11.

In addition, a cross flow fan 12 and an indoor fan motor 13 that rotatably drives the cross flow fan 12, are provided in the indoor unit 2. The cross flow fan 12 is cylindrically shaped, is provided with blades at the perimeter in the direction of the axis of rotation, and generates an air flow in a direction that intersects the axis of rotation. The cross flow fan 12 sucks indoor air into the indoor unit 2, exchanges heat with the indoor heat exchanger 11, and then blows the air out into the indoor space.

The outdoor air conditioning unit 5 comprises a compressor 21, a four-way switching valve 22 that is connected to the compressor 21 on the discharge side, an accumulator 23 that is connected to the compressor 21 on the inlet side, an outdoor heat exchanger 24 that is connected to the four-way switching valve 22, and a motor-operated valve 25 that is connected to the outdoor heat exchanger 24. The motor-operated valve 25 is connected to a passageway 31 via a filter 26 and a liquid shutoff valve 27, and is connected to one end of the indoor heat exchanger 11 via the passageway 31. In addition, the four-way switching valve 22 is connected to a passageway 32 via a gas shutoff valve 28, and is connected to the other end of the indoor heat exchanger 11 via the passageway 32. These passageways 31, 32 correspond to the refrigerant passageway 6 shown in FIG. 1

In addition, an outdoor fan 29 for externally discharging the air after exchanging heat with the outdoor heat exchanger 24 is provided in the outdoor air conditioning unit 5. This outdoor fan 29 is rotatably driven by an outdoor fan motor 30.

### CONSTITUTION OF THE OUTDOOR UNIT 3

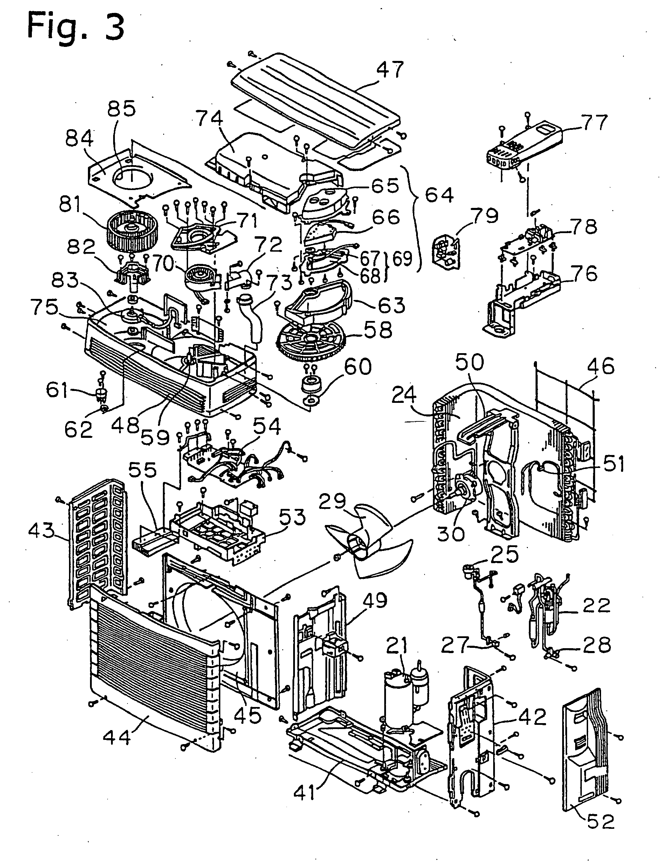

The following explains the constitution of the outdoor unit 3, referencing the exploded perspective view (FIG. 3).

The outdoor unit 3 comprises a lower outdoor air conditioning unit 5 and an upper humidifying unit 4, and is provided with an outdoor unit casing that includes a bottom plate 41, a right-side plate 42, a left-side plate 43, a front plate 44, a protective mesh 46, a top plate 47, a humidifying unit casing 48, and the like.

### CONSTITUTION OF THE OUTDOOR AIR CONDITIONING UNIT 5

A fan inlet 45 and a partition plate 49 are affixed rearward of the front plate 44. In addition, the outdoor heat exchanger 24, which is approximately L-shaped when viewed from a plan view, is affixed to the front side of the protective mesh 46, which is positioned at the rear side of the outdoor unit casing.

A fan motor mount 50, for securing the outdoor fan motor 30, is affixed to the front side of the outdoor heat exchanger 24. The outdoor fan motor 30 is provided in order to rotate the outdoor fan 29. The outdoor fan 29 provides negative pressure in the space formed by the fan inlet 45, the partition plate 49, the left-side plate 43, the outdoor heat exchanger 24, and the bottom plate of the humidifying unit casing 48. Further, the outdoor fan 29 plays the role of bringing the air introduced from the rear side and the left side of the outdoor unit casing into contact with the outdoor heat exchanger 24, and exhausts that air forward of the front plate 44.

The refrigerant circuit components, such as the compressor 21, the four-way switching valve 22, the motor-operated valve 25, the liquid shutoff valve 27, and the gas shutoff valve 28; and a thermistor 51, which detects the temperature of each part; are disposed between the partition plate 49 and the right-side plate 42. A shutoff valve cover 52 for protecting the liquid shutoff valve 27 and the gas shutoff valve 28 is affixed rightward of the right-side plate 42.

An electrical equipment box 53 is affixed above the outdoor fan 29. The electrical equipment box 53 houses a printed circuit board 54, on which is mounted electrical parts (electrical equipment) for controlling each part, and a radiating fin 55 for radiating the heat generated by the electrical parts.

### CONSTITUTION OF THE HUMIDIFYING UNIT 4

The humidifying unit 4 is provided with the humidifying unit casing 48 positioned at the upper part of the outdoor unit 3. The right side of the inside of the humidifying unit casing 48 is a space that houses a humidifying rotor 58, and the like; and the left side is an adsorbing fan housing space 75 that houses an adsorbing fan 81, and the like. The humidifying rotor 58, a heater assembly 64, a humidifying fan 70, a cover member 74, the adsorbing fan 81, and the like, are arranged in the humidifying unit casing 48.

The humidifying rotor 58 is a ceramic rotor with a honeycomb construction having an approximate disc shape, and is structured so that it can easily transit air. Specifically, as shown in FIG. 3, the rotor has a circular shape when viewed from the plan view, and has a fine honeycomb shape when viewed from a cross sectional view that cuts the horizontal plane. Further, air can pass through the numerous cylindrical parts of the humidifying rotor 58, whose cross section is a polygon.

The principal part of the humidifying rotor 58 is baked from an adsorbent material like zeolite. Although zeolite is used herein, it is also possible to use adsorbent materials like silica gel and alumina. An adsorbent material like zeolite is characterized in that it adsorbs moisture in the air that it contacts, and desorbs the adsorbed moisture upon heating.

In addition, the humidifying rotor 58 is constituted so that it supports a deodorizing catalyst that decomposes the odor of methyl sulfide, ammonia, trimethylamine, and the like, into an odorless component. Specifically, the catalyst is supported on the humidifying rotor 58 by coating the catalyst on a humidifying rotor 58 that has been impregnated with zeolite, and baked.

In contemplated embodiments not falling within the scope of Claim 1, zeolite and a catalyst are spread in an approximately uniform manner across the entire humidifying rotor 58 because the zeolite and the catalyst were included in the humidifying rotor 58 by using a solution of zeolite mixed with the catalyst.

However, according to the present invention, the catalyst is included in the humidifying rotor 58 by coating the catalyst, as noted above, whereby it is possible to position the catalyst according to the circumstances, as described below.

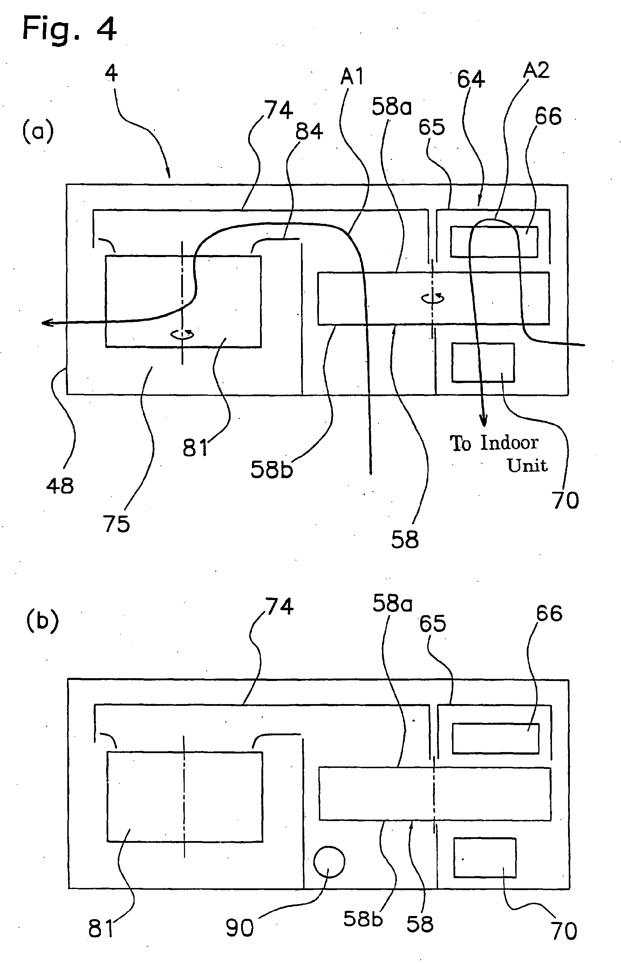

Specifically, the catalyst is coated on the upper side 58a (refer to FIG. 4) of the humidifying rotor 58 near the heater main body 66, such that the catalyst will be positioned more toward the upper part of the humidifying rotor 58. Upon doing so, the catalyst will become easily heated and, since the catalyst is of a type whose effectiveness is controlled by temperature, it is possible to increase the effectiveness of decreasing the polluting components by the catalyst.

The humidifying rotor 58 is rotatably supported via a rotor guide 60 around a support shaft 59 provided on the humidifying unit casing 48 side of the humidifying rotor 58. A gear is formed at the rim of the humidifying rotor 58, which meshes with a rotor drive gear 62 that is attached to the drive shaft of a rotor drive motor 61.

The heater assembly 64 is disposed so that it covers approximately half (the right-side half) of the top side of the humidifying rotor 58. The heater assembly 64 comprises a heater main body 66, an upper cover 65 that covers the heater main body 66, and a lower cover 69. An inlet 67 for sucking in air, and an outlet 68 for discharging air heated by the heater main body 66 to the humidifying rotor 58 side, are formed in the lower cover 69. The heater assembly 64 is attached above the humidifying rotor 58 via a heater stationary plate 63.

The humidifying fan 70 is below the humidifying rotor 58, and is disposed at a position opposing the heater assembly 64. The humidifying fan 70 is a centrifugal fan that is disposed in a space linked to a communicating duct 72 (a space below the outlet 68 of the lower cover 69 of the heater assembly 64). Further, the humidifying fan 70 is monolithically provided with a humidifying fan inlet 71, which is attached below the humidifying rotor 58. The humidifying fan 70 exhausts to the communicating duct 72 side the air that passed through the humidifying rotor 58 and came down from the near-side portion of the approximately half portion on the right side of the humidifying rotor 58 (the portion positioned below the outlet 68 of the lower cover 69 of the heater assembly 64). The air that passes out of the communicating duct 72 is supplied to the indoor unit 2 via a humidifying hose 73 and the inlet passageway 7.

A cover member 74 covers a portion (the approximately half portion on the left side) where the heater assembly 64 is not positioned on the top side of the humidifying rotor 58. The cover member 74, along with an adsorbing-side bell mouth 84 (discussed later), forms an air passageway that passes from the top side of the left half portion of the humidifying rotor 58 to the upper part of the adsorbing fan housing space 75, which is explained below.

The adsorbing fan 81, which is housed in the adsorbing fan housing space 75, is a centrifugal fan that is rotated by an adsorbing fan motor 83. Further, the adsorbing fan 81 sucks in air from an opening 85 of the adsorbing-side bell mouth 84 disposed at the upper part thereof, and exhausts the air to the outside of the adsorbing fan housing space 75 (outside of the humidifying unit casing 48). The adsorbing-side bell mouth 84 is provided at the upper part of the adsorbing fan housing space 75, and plays the role of guiding to the adsorbing fan 81 the air that passes through the air passageway formed by the cover member 74. Furthermore, an adsorbing fan motor 83 is fixed in the humidifying unit casing 48 by a motor affixing block 82.

Furthermore, a power supply board 79, an electrical equipment casing, and the like, are disposed in the humidifying unit casing 48. The electrical equipment casing comprises an electrical equipment box 76, which houses a printed circuit board 78 therein, and a cover 77.

### OPERATION OF THE HUMIDIFYING UNIT 4

In the humidifying unit 4 as described above, air from the outside is taken into the humidifying unit casing 48 by rotationally driving the adsorbing fan 81. The air that entered into the humidifying unit casing 48 passes through at approximately half portion on the left side of the humidifying rotor 58, and is exhausted outwards from the adsorbing fan housing space 75 via the adsorbing fan 81 and an air passageway formed by the cover member 74 and the adsorbing-side bell mouth 84 (refer to FIG. 4). When the air taken externally into the humidifying unit casing 48 passes through an approximately half portion on the left side of the humidifying rotor 58, the humidifying rotor 58 adsorbs the moisture included in the air.

The approximately half portion on the left side of the humidifying rotor 58, which adsorbed the moisture by the adsorbing process, becomes the approximately half portion on the right side of the humidifying rotor 58 by the rotation of the humidifying rotor 58. In other words, the adsorbed moisture moves to the portion of the humidifying rotor 58 positioned below the heater assembly 64 attendant with the rotation of the humidifying rotor 58. Further, the moisture that has moved therein is desorbed, by the heat from the heater main body 66 of the heater assembly 64 into the air flow generated by the humidifying fan 70.

When the humidifying fan 70 is rotationally driven, air is taken externally into the humidifying unit casing 48, and that air passes from below to above a far-side portion of the approximately half portion on the left side of the humidifying rotor 58, and is then introduced from the inlet 67 of the lower cover 69 into the upper cover 65. Further, the air that entered into the upper cover 65 is exhausted from the outlet 68, passes from above to below a near-side portion of the approximately half portion on the left side of the humidifying rotor 58, and then arrives at the humidifying fan 70, which generates this air flow. The humidifying fan 70 exhausts to the communicating duct 72 side the air that passed through the humidifying rotor 58, as described above, and delivers that air to the indoor unit 2 via the humidifying hose 73 and the inlet passageway 7. The air delivered to the indoor unit 2 includes the moisture that was adsorbed in the humidifying rotor 58. In addition, when the air delivered to the indoor unit 2 passes through the humidifying rotor 58, the contained odor component is decomposed by the deodorizing catalyst, changing to a state in which almost no malodor is present.

In this manner, the air supplied from the humidifying unit 4 to the indoor unit 2 is mixed into the air-conditioned air that was blown out by the cross flow fan 12 into the indoor space.

### AIR CONDITIONING OPERATION CONTROL

One control unit is arranged in each of the indoor unit 2, the outdoor air conditioning unit 5, and the humidifying unit 4, respectively. The control unit controls the operation of each motorized device in accordance with the operation mode, such as a heating operation, a cooling operation, a drying operation, a humidifying operation, and an air supplying operation.

The control unit executes the humidifying operation if a humidify command is received from a remote control (not shown), or if it is judged that the humidifying operation is needed in accordance with a humidified automatic operation command from the remote control. The humidifying operation is also often performed together with the heating operation. Inside the humidifying unit 4, the rotor drive motor 61, the heater main body 66, the motor that rotates the humidifying fan 70, and the adsorbing fan motor 83 are driven during the humidifying operation. During the humidifying operation, the moisture included in the air externally introduced into the humidifying unit 4 by the rotation of the adsorbing fan 81 is adsorbed by the humidifying rotor 58, the air that was heated by the heater main body 66 passes through the humidifying rotor 58 by the rotation of the humidifying fan 70, and the air that includes the water that was desorbed from the humidifying rotor 58 is supplied to the indoor unit 2.

During the humidifying operation, heating by the heater main body 66 is controlled so that the a surface 58a (refer to FIG. 4 (a)) on the heater side of the humidifying rotor 58 reaches 100°C to 300°C. The purpose of heating control lies in adsorbing the moisture from the humidifying rotor 58.

In addition, if it is desired to supply outdoor air from the humidifying unit 4 to the indoor unit 2 for the purpose of ventilating the indoor space, then the control unit performs the air supplying operation. In the air supplying operation, the outdoor air is supplied to the indoor unit 2 by rotating just the humidifying fan 70, without rotating the adsorbing fan 81. When performing this type of air supplying operation, the outdoor air, which in principal is taken in from outside of the humidifying unit 4, is supplied to the indoor unit 2 as is, without humidifying and without the moisture being newly adsorbed in the humidifying rotor 58. However, because the deodorizing catalyst decomposes the odor component in the air when that air passes through the humidifying rotor 58, the air supplied to the indoor unit 2 includes almost no odor component.

During the air supplying operation, the heating of the humidifying rotor 58 by the heater main body 66 is controlled so that the temperature reaches a level wherein the decomposition (chemical reaction) of the odor component by the deodorizing catalyst included in the humidifying rotor 58 is promoted. Specifically, control is performed so that the electric power value flowing to the heater main body 66 becomes smaller during the air supplying operation than during the humidifying operation.

### CHARACTERISTICS OF THE HUMIDIFYING ROTOR 58 AND THE AIR CONDITIONER 1

**(1)**
   Zeolite for adsorbing and desorbing moisture, and a deodorizing catalyst for decomposing the odor component in the air are included in a singular humidifying rotor 58. In this manner, because the humidifying rotor 58 is monolithically provided with zeolite, which is a moisture adsorbing and desorbing material, and a deodorizing catalyst, which decomposes odors, it is no longer necessary to separately provide a filter and the like for eliminating odors, and an increase in the size of the humidifying unit 4 of the air conditioner 1 can be controlled.
   Because the air supplied to the indoor unit 2 by the rotation of the humidifying fan 70 of the humidifying unit 4 passes through the humidifying rotor 58, most of the odor component in the air is decomposed and extinguished (changed to an odorless component) when passing therethrough. Accordingly, even in a case where the outdoor unit 3 is placed near a gutter in which wastewater is flowing, the introduction of malodor into the indoor space is controlled by performing the humidifying operation or the air supplying operation.
   Furthermore, the deodorizing catalyst itself hardly deteriorates merely by promoting a chemical reaction that changes the malodor component into an odorless component. Accordingly, the effectiveness of the humidifying rotor 58 in decomposing the malodor can be sustained for a long time.
**(2)**
   The air conditioner 1 controls the heater main body 66 so that the humidifying function of the humidifying rotor 58 is sufficiently produced during the humidifying operation, and so that the function of the humidifying rotor 58 in decomposing the odor component is sufficiently produced during the air supplying operation. In this manner, because control of the heater main body 66 is divided into two operations, the humidifying operation and the air supplying operation, the effectiveness of the humidifying rotor 58 is appropriately produced in the air conditioner 1.

### OTHER EMBODIMENTS

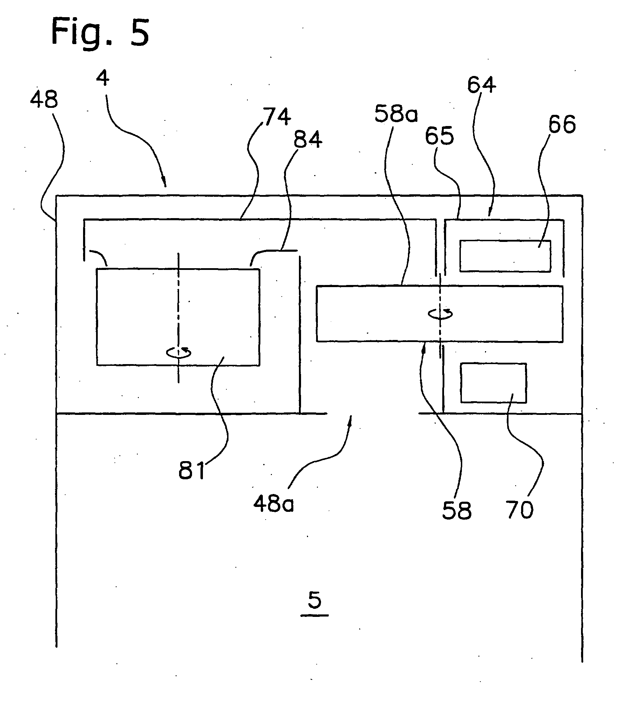

**(A)**
   In the abovementioned embodiment, the deodorizing catalyst that decomposes the odor of methyl sulfide, ammonia, trimethylamine, and the like, into an odorless component is supported on the humidifying rotor 58; however, in place of such a deodorizing catalyst (or, in addition to the deodorizing catalyst), it is also conceivable to support a catalyst on the humidifying rotor 58, wherein the catalyst changes deleterious components in the air into innocuous components. For example, it is conceivable to combine various catalysts that change dioxin, formaldehyde, and the NOx, SOx, CO, HC, and the like, which are included in the exhaust gas of automobiles and the like, into innocuous substances (innocuous components). If such catalysts are supported on the humidifying rotor 58, outdoor air can be taken into the indoor space by operating the humidifying unit 4 of the outdoor unit 3, and performing the humidifying operation or the air supplying operation, even if located near an arterial road or a high-speed highway, and the outdoor air includes a large amount of exhaust gas from automobiles. Although it is problematic to open the window and allow natural ventilation if the ambient air needs cleaning, ventilation can be performed effectively by the use of this humidifying unit 4.
(B)
   In the abovementioned embodiment, the humidifying rotor 58 was rotated; however, it is also conceivable to use an element, without rotating it, that includes a moisture adsorbing and desorbing material (zeolite) and a catalyst.
(C)
   In the abovementioned embodiment, the humidity regulating element (humidifying rotor 58) according to the present invention is applied to a stand-alone air conditioner 1; however, it is also possible to apply the humidity regulating element to an indoor/outdoor integrated air conditioner, an apparatus specialized for dehumidifying that is placed in the indoor space, an apparatus specialized for humidifying, and the like.
(D)
   For the catalyst included in the humidifying rotor 58, it is also effective to use a so-called photocatalyst, which turns deleterious components in the air into innocuous ones by the impingement of ultraviolet light. Titanium oxide, which is a currently used photocatalyst, is impinged by the energy of the ultraviolet light, generates active oxygen (hydroxy radical, superoxide anion), and decomposes organic substances and inorganic substances by the action of that active oxygen.
   If such a photocatalyst is included in the humidifying rotor 58, then it is preferable to arrange the lamp 90, which irradiates ultraviolet light, below the humidifying rotor 58, as shown in FIG. 4 (b). Because the humidifying rotor 58 is covered by the humidifying unit casing 48, the humidifying rotor 58 does not receive much external light (sun rays, reflected light, etc.). If the humidifying rotor 58, which includes the photocatalyst, is impinged by the ultraviolet light from the lamp 90, it is possible to sufficiently produce the function of the humidifying rotor 58, i.e., to decrease the amount of polluting components in the air.
   In addition, if a photocatalyst is included in the humidifying rotor 58, then it is also conceivable to form an opening 48a in the humidifying unit casing 48 instead of providing the abovementioned lamp 90. As shown in FIG. 5, the opening 48a links the space below the humidifying rotor 58 in the humidifying unit 4 and the space in the outdoor air conditioning unit 5. Thus, if an opening 48a is provided, the humidifying rotor 58 can also be impinged, via the opening 48a, by the ultraviolet light entering into the outdoor air conditioning unit 5, and the function of the photocatalyst in the humidifying rotor 58 can be sufficiently produced.
   Furthermore, because the humidifying rotor 58, which includes a photocatalyst, is provided in the outdoor unit 3 instead of in the indoor unit 2, much ultraviolet light included in natural light, sun rays, and the like, can impinge on the photocatalyst of the humidifying rotor 58.
(E)
   The abovementioned embodiment used the present invention in the air conditioner 1 comprising the humidifying unit 4 that takes in outdoor air and supplies that air to the indoor space; however, the present invention can be used in an apparatus that takes in indoor air and supplies that air once again to the indoor space. Even in this case, the deleterious components, such as malodorous components, present in the indoor space decrease by the passage of air through an element (the member corresponding to the humidifying rotor 58), and the apparatus therefore does not cause the user discomfort.

### INDUSTRIAL FIELD OF APPLICATION

The use of the humidity regulating element, which monolithically comprises a catalyst and a moisture adsorbing and desorbing material, enables the polluting components in the air to be decreased by passing air through a humidity regulating element while controlling an increase in the size of the air conditioner, without providing a separate filter and the like.

## Claims

1. A humidifying unit (4) of an air conditioner (1) for adding humidified air to the regulated air supplied to an indoor space, comprising:
a humidity regulating element (58) monolithically comprising a moisture adsorbing and desorbing material for adsorbing and desorbing moisture from and to the air, and a catalyst for decreasing polluting components in the air;
a heater (66) that is arranged to heat said humidity regulating element; and
a fan (70) that is arranged to pass air through said humidity regulating element, **characterised in that**
said catalyst is provided as a coating only on a portion of said humidity regulating element (58) facing said heater (66), the effectiveness of said catalyst being increased by heating.

2. The humidifying unit (4) of the air conditioner (1) as recited in Claim 1, wherein
said humidifying unit (4) is a unit that is arranged to take in air from the outdoor space, and to supply that air to the indoor space;
said air conditioner (1) is arranged to perform a humidifying operation, wherein humidified air from said humidifying unit (4) is supplied to the indoor space, and an air supplying operation, wherein non-humidified air from said humidifying unit (4) is supplied to the indoor space; and
said heater (66) is arranged to execute a first heating control mode for desorbing moisture from said humidity regulating element (58) when in said humidifying operation, and to execute a second heating control mode for promoting the function of said catalyst of said humidity regulating element (58) when in said air supplying operation.

3. An air conditioner (1) comprising the humidifying unit (4) as recited in Claim 1 or 2, wherein
said air conditioner (1) further comprises an indoor unit (2) and an outdoor unit (3);
said humidity regulating element (58) is provided in said outdoor unit (3); and
at least a portion of said catalyst is a photocatalyst.

## Patentansprüche

1. Befeuchtungseinheit (4) einer Klimaanlage (1) zum Hinzufügen von befeuchteter Luft zu der geregelten Luft, die einem Innenraum zugeführt wird, mit:
einem Feuchtigkeitssteuerelement (58), das einstückig ein Feuchtigkeitsadsorptions- und -desorptionsmaterial zum Adsorbieren und Desorbieren von Feuchtigkeit von und zu der Luft und einen Katalysator zum Verringern von Verschmutzungskomponenten in der Luft aufweist,
einer Heizeinrichtung (66), die angeordnet ist, um das Feuchtigkeitssteuerelement zu erhitzen, und
einem Ventilator (70), der angeordnet ist, um Luft durch das Feuchtigkeitssteuerelement hindurchzuleiten, **dadurch gekennzeichnet, dass**
der Katalysator als eine Beschichtung nur auf einem Abschnitt des Feuchtigkeitssteuerelements (58), welcher der Heizeinrichtung (66) zugewandt ist, vorgesehen ist, wobei die Effizienz des Katalysators durch Erhitzen erhöht wird.

2. Befeuchtungseinheit (4) der Klimaanlage (1) nach Anspruch 1, bei der
die Befeuchtungseinheit (4) eine Einheit ist, die dafür angeordnet ist, Luft aus dem Außenraum aufzunehmen und diese Luft dem Innenraum zuzuführen,
bei der die Klimaanlage (1) dafür angeordnet ist, einen Befeuchtungsbetrieb, bei dem befeuchtete Luft aus der Befeuchtungseinheit (4) dem Innenraum zugeführt wird, und einen Luftzuführbetrieb durchzuführen, bei dem nicht befeuchtete Luft aus der Befeuchtungseinheit (4) dem Innenraum zugeführt wird, und
bei der die Heizeinrichtung (66) angeordnet ist, um einen ersten Heizregelmodus zum Desorbieren von Feuchtigkeit von dem Feuchtigkeitssteuerelement (58) durchzuführen, wenn es sich in dem Befeuchtungsbetrieb befindet, und um einen zweiten Heizregelmodus zum Fördern der Funktion des Katalysators des Feuchtigkeitssteuerelements (58) durchzuführen, wenn es sich in dem Luftzuführbetrieb befindet.

3. Klimaanlage (1) mit der Befeuchtungseinheit (4) nach Anspruch 1 oder 2, bei der
die Klimaanlage (1) ferner eine Inneneinheit (2) und eine Außeneinheit (3) aufweist,
bei der das Feuchtigkeitssteuerelement (58) in der Außeneinheit (3) vorgesehen ist und
bei der wenigstens ein Teil des Katalysators ein Fotokatalysator ist.

## Revendications

1. Unité d'humidification (4) d'un climatiseur (1) pour ajouter de l'air humidifié à l'air régulé fourni à un espace intérieur, comprenant :
un élément de régulation de l'humidité (58) comprenant de manière monolithique un absorbant d'humidité et un matériau de désorption pour adsorber et désorber l'humidité de l'air et vers l'air, et un catalyseur pour réduire les composants polluants dans l'air ;
un chauffage (66) qui est disposé pour chauffer ledit élément de régulation de l'humidité ; et
un ventilateur (70) qui est disposé pour faire passer l'air à travers ledit élément de régulation de l'humidité, **caractérisé en ce que**
ledit catalyseur est prévu comme un revêtement sur une partie seulement dudit élément de régulation de l'humidité (58) en vis-à-vis dudit chauffage (66), l'efficacité dudit catalyseur étant augmentée par chauffage.

2. Unité d'humidification (4) du climatiseur (1) selon la revendication 1, dans lequel
ladite unité d'humidification (4) est une unité qui est disposée pour prendre l'air à partir de l'espace extérieur, et pour fournir cet air à l'espace intérieur ;
ledit climatiseur (1) est disposé pour effectuer une opération d'humidification, où l'air humidifié provenant de ladite unité d'humidification (4) est fourni à l'espace intérieur, et une opération d'alimentation en air, où de l'air non humidifié provenant de ladite unité d'humidification (4) est fourni à l'espace intérieur ; et
ledit chauffage (66) est disposé pour exécuter un premier mode de commande de chauffage pour désorber l'humidité à partir dudit élément de régulation de l'humidité (58) pendant ladite opération d'humidification, et pour exécuter un second mode de commande de chauffage pour favoriser la fonction dudit catalyseur dudit élément de régulation de l'humidité (58) pendant ladite opération d'alimentation d'air.

3. Climatiseur (1) comprenant l'unité d'humidification (4) selon la revendication 1 ou 2, dans lequel
ledit climatiseur (1) comprend en outre une unité intérieure (2) et une unité extérieure (3) ;
ledit élément de régulation de l'humidité (58) est prévu dans ladite unité extérieure (3) ; et
au moins une partie dudit catalyseur est un photo-catalyseur.
